# EUROPEAN PATENT APPLICATION

(11) **EP 2 327 403 A2**
(43) Date of publication of application: **01.06.2011**
(21) Application number: 11158508.9
(22) Date of filing: 19.02.2008
(51) Int. Cl.: A61K 31/137, A61K 31/167, A61K 31/439, A61K 31/46, A61K 31/4704, A61K 31/573, A61K 31/58, A61K 45/06, A61P 11/00

(54) **Pharmaceutical combinations of at least two bronchodilators**

(30) Priority: 19.02.2007 IN MU03142007; 27.08.2007 IN MU16422007; 01.11.2007 IN MU21792007
(62) Divisional of application: 08709463.7
(71) Applicant: Cipla Limited, Mumbai 400 008 (IN)
(72) Inventor: Lulla, Amar, 400 005 Mumbai (IN); Malhotra, Geena, 400 010 Mumbai (IN)
(74) Representative: Turner, Craig Robert

(57) **Abstract**

A pharmaceutical combination comprising a combination of two or more bronchodilators.

## Description

### Technical field:

The present invention relates to combinations of inhalable medicaments for simultaneous or sequential administration in the prevention or treatment of respiratory, inflammatory or obstructive airway disease.

### Background & Prior art:

Chronic obstructive pulmonary disease (COPD) is a preventable and treatable disease state characterized by airflow limitation that is not fully reversible.

The airflow limitation is usually progressive and is associated with an abnormal inflammatory response of the lungs to noxious particles or gases, primarily caused by cigarette smoking.

It is expected to be the third leading cause of death by 2020. Approximately 14 million Indians are currently suffering form COPD. Currently there are 94 million smokers in India. 10 lakh Indians die in a year due to smoking related diseases

Although COPD affects the lungs, it also produces significant systemic consequences.

Various therapies are currently used for the treatment of COPD. Bronchodilators and steroids form the major classes of drugs used.

WO0139745 discloses dry powder inhalation comprising formoterol and its salts or derivatives thereof and pharmaceutically acceptable particulate diluent.

A combination therapy is described in patent number WO0176601, using short acting anticholinergic ipratropium bromide and long acting β-agonist salmeterol are.

A further combination therapy, using steroid along with anticholinergics and beta agonist, has been described in US6423298 and WO0207672.

WO0207672 relates to a medicinal aerosol formulation and more particularly, to a medicinal aerosol formulation containing a particulate drug, or combination of at least two particulate drugs a propellant and a stabilizing agent comprising water. The medicament is selected from the group consisting of albuterol, atropine, budesonide, cromolyn, epinephrine, ephedrine, fentanyl, flunisolide, formoterol, ipratropium bromide, isoproterenol, pirbuterol, prednisone, mometasone, triamcinolone acetonide, salmeterol, amiloride, fluticasone, (-) 4-amino-3,5-dichloro-a- [ [ [6 (2pyridinyl) ethoxy] hexyl] amino] methyl] benzene-methanol and pharmaceutically acceptable salts, esters, hydrates and solvates of the foregoing.

US6423298 relates to pharmaceutical formulations for aerosols with at least two or more active substances for administration by inhalation or by nasal route.

WO2004019985 discloses certain other drug pharmaceutical formulations for aerosols.

Various other combinations are known in the art. But a problem associated with these drugs is that not all are once a day formulations and also if some are, they require an additional dose of a beta agonist to substantiate the combination.

Even from the patient compliance point of view, the treatment calls for the patient to comply with different dosage regimens, different frequencies of administration, etc. Also, since most of the medications are in the form of aerosols, the patient is required to carry several formulations and dispensers.

Thus there remains a need to formulate and also offer to the patient a combination that needs to be taken once a day and where the patient does not have to take another dose of beta-agonist or any other to substantiate the combination dose.

### Object of the Invention:

It is an object of the present invention is to provide combinations of inhalable medicaments for administration in the prevention or treatment of respiratory, inflammatory or obstructive airway disease.

It is another object of the present invention to provide combinations of inhalation medicaments for administration once daily.

It is yet another object of the present invention to provide a method of formulating such novel combinations.

### Summary:

According to a first aspect of the present invention there is provided a pharmaceutical combination comprising at least two active substances for once daily administration by inhalation route.

According to another aspect of the present invention there is provided a method of manufacturing said pharmaceutical combination comprising at least two active substances for once daily administration by inhalation route.

According to still another aspect of the present invention there is provided a pharmaceutical combination comprising at least two active substances for once daily administration by inhalation route administration in the prevention or treatment of respiratory, inflammatory or obstructive airway disease.

### Description:

As discussed above, there is a long felt want in the art for an effective once daily formulation. The present invention provides novel combinations comprising two or more actives for administration once daily.

The actives may be selected from various classes consisting of bronchodilators and .. corticosteroids and mixtures thereof. The bronchodilators may be either beta-agonists or anticholinergics or both.

The present inventors have surprisingly found that a combination of two or more bronchodilators and / or combination of two or more bronchodilators in combination with corticosteroids shows a synergistic effect by relaxing both the central and peripheral air pathways improving the airflow obstruction, prolonged bronchodilation and provides rapid onset of action with reduced exacerbations.

Pharmaceutically active agents useful in the present invention include one or more of drugs selected from various classes consisting of bronchodilators and corticosteroids and mixtures thereof. The bronchodilators may be beta-agonists and/or anticholinergics. The terms "beta-agonist agent" or "beta-agonist" or "anticholinergic agent" or "corticosteroids" are used in broad sense to include not only the beta-agonist or anticholinergic agent per se but also their pharmaceutically acceptable salts, pharmaceutically acceptable solvates, pharmaceutically acceptable hydrates, pharmaceutically acceptable enantiomers, pharmaceutically acceptable derivatives, pharmaceutically acceptable polymorphs, pharmaceutically acceptable prodrugs, etc.

The beta-agonists may be selected from formoterol, AR-formoterol, fenoterol, carmoterol, indacaterol and the like. Formoterol may be present in an amount ranging from about 4.5- 96 mcg, AR-formoterol may be present in an amount ranging from 1.25-96 mcg, carmoterol may be present in an amount ranging from 0.5-16 mcg, and indacaterol may be present in an amount ranging from 25-800 mcg.

The anticholinergic may be aclidinium, ipratropium, tiotropium, oxitropium and the like. Tiotropium may be present in an amount ranging from 4.5 - 160 mcg. Aclidinium may be present in an amount ranging from 50 - 900 mcg.

The corticosteroid may be budesonide, ciclesonide, mometasone, beclomethasone and the like. Ciclesonide may be present in an amount ranging from 40-2880 mcg and mometasone present in an amount ranging from 25-2000 mcg.

In one preferred embodiment of the present invention, the beta agonist is carmoterol, the corticosteroid is ciclesonide and the anticholinergic is tiotropium. In another preferred embodiment of the present invention, the beta agonist is carmoterol and the corticosteroid is ciclesonide. In yet another preferred embodiment of the present invention, the anticholinergic is tiotropium and the beta agonist is carmoterol. In yet another preferred embodiment of the present invention, the anticholinergic is aclidinium and the beta agonist is formoterol and/or AR-formoterol.

The formulations of the present invention are suitable for use in metered dose inhalers (MDI) (or aerosols). MDIs are compact drug delivery systems that use a liquefied propellant to atomize a precisely metered volume of a pharmaceutical formulation into particles, which are small enough to penetrate deep into the patient's lungs. MDIs allow for targeted delivery of drug to the desired site of the therapeutic effect - the lung.

The pharmaceutical combination may further be combined with one or more pharmaceutically acceptable excipients in order to provide a suitable formulation. The combination may, for example, be formulated as a propellant free inhalation solution for nebulization, as an aerosol composition (with propellant), or as dry powder composition for inhalation.

In case of dry powder inhalation formulations, the drugs may be used alone or optionally together with a finely divided pharmaceutically acceptable carrier, which is preferably present and may be chosen from materials known as carriers in dry powder inhalation compositions, for example saccharides, including monosaccharides, disaccharides, polysaccharides; and sugar alcohols such as arabinose, glucose, fructose, ribose, mannose, sucrose, trehalose, lactose, maltose, starches, dextran or mannitol.

Preferably, the pharmaceutically acceptable carrier in the dry powder inhalation formulation is lactose. The carrier is preferably present in an amount not less that 75% by weight of the formulation.

The dry powder may be provided in capsules of gelatin or HPMC or in blisters or alternatively, the dry powder may be contained as a reservoir either in a single dose or multi-dose dry powder inhalation device. The particle size of the active ingredient and that of the carrier where present in dry powder compositions, can be reduced to the desired level by conventional methods, for example by grinding in an air-jet mill, ball mill or vibrator mill, microprecipitation, spray-drying, lyophilization or recrystallization from supercritical media.

According to a preferred embodiment, the above dry powder composition may be manufactured by any convenient means wherein the process comprises of two or more drugs being mixed optionally with a pharmaceutically acceptable carrier and providing the ingredients in a suitable dry powder inhaler.

In case of inhalation solutions or nebulizing solutions, the drugs may be combined with suitable excipients such as wetting agents, tonicity adjusting agents, pH regulators, buffering agents and chelating agents, in a suitable vehicle. The wetting agents may be selected from the group consisting of sodiumdioctylsulfosuccinate; polysorbates such as polysorbate 20, polysorbate 40, polysorbate 60, polysorbate 80, polysorbate 65, polysorbate 85; sorbitan fatty acid esters such as Span 20, Span 40, Span 60 Span 80, Span 120; sodium lauryl sulfate; polyethoxylated castor oil; polyethoxylated hydrogenated castor oil and the like. The preferred wetting agent is one or more polysorbates, either alone or in combination with another wetting agent. The amount of wetting agent (especially when it includes a polysorbate) is in a range of 0.001 to 0.1 % w/v of the composition.

The tonicity-adjusting agent, which may be used in the present invention, may include one or more of sodium chloride, potassium chloride, zinc chloride, calcium chloride and mixtures thereof. The preferred tonicity adjusting agent is sodium chloride.

The pH regulator may be selected from pharmacologically acceptable inorganic acids or organic acids, such as sodium hydroxide, sodium carbonate, sodium bicarbonate, magnesium carbonates, aluminium hydroxide, aluminum carbonate, aluminium bicarbonate, calcium carbonate and calcium hydroxide, calcium bicarbonate and the like. The preferred inorganic acids are selected from the group consisting of hydrochloric acid, hydrobromic acid, nitric acid, sulphuric acid, phosphoric acid and the like. The preferred organic acids are selected from the group consisting of ascorbic acid, citric acid, malic acid, tartaric acid, maleic acid, succinic acid, fumaric acid, acetic acid, formic acid and propionic acid. The most preferred inorganic acids are hydrochloric acid & sulphuric acid, while, for the organic acids, ascorbic acid, citric acid and fumaric acid are the most preferred.

The pH regulators may be selected from pharmacologically acceptable inorganic bases or organic bases. The preferred inorganic bases are selected from the group consisting of sodium hydroxide, potassium hydroxide, ammonium hydroxide, sodium carbonate, calcium hydroxide. The preferred organic bases are selected from the group consisting of methyl amine, ethyleneimine, hydroquinone, ethylamine, dimethylamine, ethanolamine, butylamine, diethylamine. The most preferred base is sodium hydroxide.

Preferably a nasal inhalation formulation as provided by the present invention has a pH in the range of 3.0 to 7.0.

In a preferred embodiment, suitable chelating or complexing agents are used in the inhalation solutions, and may be molecules which are capable of entering into complex bonds. Preferable those compounds should have the effect of complexing cations most preferably metal cations. The formulations according to the invention preferably contain EDTA or one of the known salts thereof, e.g. sodium EDTA or disodium EDTA dihydrate, as complexing agent. The preferred agent is ethylenediaminetetraacetic acid (EDTA) or a salt thereof, such as the disodium salt.

In a preferred embodiment, suitable buffering agents that may be used in the inhalation solutions include citric acid and sodium or potassium citrates; or phosphates like trisodium phosphate, disodium or trisodium hydrogen phosphate, sodium dihydrogen phosphate, potassium hydrogen phosphate, potassium dihydrogen phosphate, tripotassium hydrogen phosphate and the like.

Suitable liquid vehicles for use in the compositions of the invention (particularly inhalation solutions or suspensions) include polar solvents, such as compounds that contain hydroxyl groups or other polar groups. Such solvents may include water or alcohols, such as ethanol, isopropanol, and glycols including propylene glycol, polyethylene glycol, polypropylene glycol, glycol ether, glycerol and polyoxyethylene alcohols.

Suitable polar solvents also include protic solvents, such as water, one or more aqueous saline solutions with one or more pharmaceutically acceptable salt(s), one or more alcohols, one or more glycols or a mixture thereof. For a saline solution as the solvent or as a component thereof, particularly suitable salts are those which display no or only negligible pharmacological activity after administration.

An anti-microbial preservative agent may be added for multi-dose packages. Suitable preservatives will be apparent to the skilled person, particularly benzalkonium chloride or benzoic acid or benzoates such as sodium benzoate, sorbic acid or sorbates such as potassium sorbates in the concentration known from the prior art. The most preferred preservative is benzalkonium chloride.

According to a preferred embodiment, the above inhalation or nebulizer composition may be manufactured by any convenient means wherein the process comprises dissolving two or more drugs and optionally chelating agents, tonicity adjusting agents and any other suitable ingredient in a vehicle and adjusting the pH using a suitable pH adjusting agent. Further, finally, providing the said composition in a suitable dispensing container (such as a nebulizer) for inhalation.

According to another preferred embodiment, the above composition can be manufactured by convenient means wherein the process comprises sterilization of the active/s by known processes such as dry heat sterilization, moist heat sterilization, gamma radiation. The sterilized active/s is/are placed in a container and sterile bulk of other pharmaceutically acceptable ingredients comprising one or more of wetting agents, tonicity adjusting agents, pH regulators, chelating agents, buffering agents are transferred aseptically in the container with the API. The mixture may then be subjected to mixing either by sonication or magnetic stirring or by other means known in the art and finally, adding the above solution to the remaining bulk.

In case of formulations which are in the form of pressurized aerosols using MDIs, the formulations may comprise one or more of cosolvents, antioxidants, surfactants, bulking agents and lubricants, in addition to the actives and propellant.

In one embodiment of the present invention the actives may be combined with one or more propellants.

The present invention includes at least one propellant such as propellant 11 (dichlorodifluoromethane), propellant 12 (monofluorotrichloromethane), Propellant 114, 1,1,1,2-tetrafluoroethane, 1,1,1,2,3,3,3-heptafluoroethane

In one embodiment of the present invention the actives may be combined with either propellant 11 or propellant 114 or a combination thereof of and propellant 12 with surface-active agents known in the art.

The surfactants may be selected from the vast class known in the art like oils such as corn oil, olive oil, cottonseed oil and sunflower seed oil, mineral oils like liquid paraffin, oleic acid and also phospholipids such as lecithin, or sorbitan fatty acid esters like sorbitan trioleate or Tween 20, Tween 60, Tween 80, PEG - 25 Glyceryl trioleate, PVP, citric acid, PFDA (per fluoro-n-decanoic acid) The use of surfactants leads to comparatively good suspension quality. One of the preferred surfactants is lecithin. The surfactant can be used in a concentration of 0.001-100%. Preferably in a range of 1% - 50%. More preferably in a concentration of 5% - 30%. These concentrations are based on the weight of the active material(s).

In another embodiment of the present invention the actives and the surfactant can be micro-milled with propellant, preferably propellant 11 or propellant 114 or a combination thereof, to form a slurry and then the slurry can be filled in canisters. The actives can be micro-milled separately or together with the propellant. Micro-milling can be done to improve the suspension quality which inurn results in a better FPD.

There is also provided by the present invention a method for the manufacture of CFC aerosol which process comprises: (a) optionally micro- milling the drugs and surfactant with a propellant, preferably either propellant 11 or propellant 114 or a combination thereof; (b) filling the slurry in the canisters; (c) crimping with a suitable valve; and (d) charging with a propellant, preferably propellant 12, through the valve.

In yet another embodiment of the present invention the aerosol composition may comprise actives and either 1,1,1,2-tetrafluoroethane (HFA134a) or 1,1,1,2,3,3,3-heptafluoroethane (HFA227) or a combination thereof.

Another embodiment of the present invention may comprise actives, propellant and a cosolvent. In such a case the cosolvent has a greater polarity than the propellant. Typically the cosolvent is present in a proportion of 0.2 to 20% by weight of the total formulation. The cosolvent used may be selected from the group consisting of glycols, particularly propylene glycol, polyethylene glycol and glycerol or ethanol and mixtures thereof. Typically the cosolvent is ethanol.

The present invention also provides a method for the manufacture of the above composition which method comprises (a) Addition of the active ingredients to the canister. (b) Addition of the cosolvent optionally to (a) and sonication of the same. (c) Crimping the canister with the metered valve (d) charging the canister with the propellant.

In yet another embodiment the present invention may comprise the actives, propellant, surface-active agent and cosolvent.

The surface-active agent stabilizes the formulation and helps in the lubrication of a valve system in the inhaler. Some of the most commonly used surface active agents are those known in the art and can be selected from among various polysorbates such as Polysorbate 20, Polysorbate 40, Polysorbate 80; Acetylated monoglycerides like Myvacet 9-45 and Myvacet 9-08; isopropylmyristate, oleic acid, Polyoxyethylene ethers, ethyloleate, glyceryl trioleate, glyceryl monolaurate, glyceryl monooleate, glyceryl monosterate, glyceryl monoricinoleate, cetylalcohol, sterylalcohol, cetylpyridinium chloride, block polymers, natural oils, polyvinyl pyrrolidone, sorbitan fatty acid esters such as sorbitan trioleate, polyethoxylated sorbitan fatty acid esters (for example polyethoxylated sorbitan trioleate), sorbimacrogol oleate, synthetic amphotensides (tritons), ethylene oxide ethers of octylphenolformaldehyde condensation products, phosphatides such as lecithin, polyethoxylated fats, polyethoxylated oleotriglycerides and polyethoxylated fatty alcohols.

The surface-active agents are preferably used in a concentration of 0.02 - 10% by weight of the active ingredients.

The present invention also provides a method for the manufacture of the above composition which method comprises: (a) addition of the active ingredients to the canister; (b) addition of the cosolvent and the surfactant solution to (a) and sonication of the same; (c) crimping the canister with a metered valve; and (d) charging the canister with the propellant.

Another embodiment of the present invention comprises the actives along with bulking agent and propellant.

The bulking agent acts as a carrier for the drug to reach the lungs. The bulking agent may be present in a concentration of 10-500% by weight of the active. More preferably in a range of 10-300% by weight of the active. The bulking agent may be selected from the class of saccharides, including monosaccharides, disaccharides, polysaccharides; and sugar alcohols such as arabinose, glucose, fructose, ribose, mannose, sucrose, trehalose, lactose, maltose, starches, dextran or mannitol. The preferred bulking agent is lactose.

The present invention also provides a method for the manufacture of the above aerosol composition which method comprises: (a) addition of the active ingredients to the canister; (b) addition of the bulking agent to (a); (c) crimping the canister with a metered valve; and (d) charging the canister with the propellant.

Yet another embodiment of the present invention comprises the actives along with surfactant and propellant.

The surfactant may be selected from the class of salts of stearic acids or esters such as ascorbyl palmitate, isopropyl myristate and tocopherol esters. The surfactant is preferably the magnesium salt of stearic acid or isopropyl myristate. Most preferably the surfactant is magnesium stearate. The surfactant is preferably used in a concentration of 0.01% to 10% by weight of the active.

The present invention also provides a method for the manufacture of the above aerosol composition which method comprises: (a) addition of the active ingredients to the canister; (b) addition of the surfactant to (a); (c) crimping the canister with a metered valve; and (d) charging the canister with the propellant.

In yet another embodiment one or more actives can be spray-coated or spray-dried or coprecipitated. The spray coating, spray drying or co-precipitation may be done by mixing the active in a solution of surface active agents in a suitable non-polar solvent and further removing the solvent.

In a further aspect of the present invention there is provided a method for the treatment in a mammal, such as a human, of chronic obstructive pulmonary disease, which method comprises administration of a therapeutically effective amount ranging from a pharmaceutical composition according to the present invention.

The MDI formulations may be in plain aluminium cans or SS (stainless steel) cans. Some aerosol drugs tend to adhere to the inner surfaces, i.e., walls of the cans and valves, of the MDI. This can lead to the patient getting significantly less than the prescribed amount of the active agent upon each activation of the MDI. Coating the inner surface of the container with a suitable polymer can reduce this adhesion problem. Suitable coatings include fluorocarbon copolymers such as FEP-PES (fluorinated ethylene propylene and polyethersulphone) and PFA-PES (perfluoroalkoxyalkane and polyethersulphone), epoxy and ethylene.

Alternatively, the inner surfaces of the cans may be anodized.

The following examples are for the purpose of illustration of the invention only and are not intended in any way to limit the scope of the present invention.

It will be readily apparent to one skilled in the art that varying substitutions and modifications may be made to the invention disclosed herein without departing from the scope of the invention. Thus, it should be understood that although the present invention has been specifically disclosed by the preferred embodiments and optional features, modification and variation of the concepts herein disclosed may be resorted to by those skilled in the art, and such modifications and variations are considered to be falling within the scope of the invention.

### Example 1

An aerosol formulation was prepared using formoterol and aclidinium or a salt thereof, preferably aclidinium bromide, with a propellant, which is preferably HFA P227. The amounts of the materials is preferably as shown in the table:

| Ingredients | Qty/Can |
|---|---|
| Formoterol | 0.96 mg |
| Aclidinium Bromide | 32 mg |
| Propellant HFA P227 | 11.2 gm |

### Example 2

An aerosol formulation was prepared using formoterol and aclidinium or a salt thereof, preferably aclidinium bromide, with lactose and a propellant, which is preferably HFA 134a. The amounts of the materials is preferably as shown in the table:

| Ingredients | Qty/Can |
|---|---|
| Formoterol | 0.96 mg |
| Aclidinium Bromide | 32mg |
| Lactose | 38 mg |
| Propellant HFA134a | 9.6 g |

### Example 3

An aerosol formulation was prepared using formoterol and aclidinium or a salt thereof, preferably aclidinium bromide (spray coated with PVP), with lactose and a propellant, which is preferably HFA P227. The amounts of the materials is preferably as shown in the table:

| Ingredients | Qty/Can |
|---|---|
| Formoterol | 0.96 mg |
| Aclidinium Bromide (spray coated with PVP) | 32 mg |
| Lactose | 3 8 mg |
| Propellant P227 | 11.2 g |

### Example 4

An aerosol formulation was prepared using formoterol and aclidinium or a salt thereof, preferably aclidinium bromide, with PEG 100 and a propellant, which is preferably HFA 134a. The amounts of the materials is preferably as shown in the table:

| Ingredients | Qty/Can |
|---|---|
| Formoterol | 0.96 mg |
| Aclidinium Bromide | 16 mg |
| PEG 100 | 0.0288 g |
| Propellant HFA 134a | 9.6 g |

### Example 5

An aerosol formulation was prepared using formoterol and aclidinium or a salt thereof, preferably aclidinium bromide, with absolute alcohol, oleic acid, and a propellant, which is preferably HFA 134a. The amounts of the materials is preferably as shown in the table:

| Ingredients | Qty/Can |
|---|---|
| Formoterol | 0.96 mg |
| Aclidinium Bromide | 16 mg |
| Absolute Alcohol | 1.44 g |
| Oleic acid | 0.11 mg |
| Propellant HFA134a | 8.16 g |

### Example 6

An aerosol formulation was prepared using formoterol and aclidinium or a salt thereof, preferably aclidinium bromide, with absolute alcohol, lecithin and a propellant, which is preferably HFA 134a. The amounts of the materials is preferably as shown in the table:

| Ingredients | Qty/Can |
|---|---|
| Formoterol | 0.96 mg |
| Aclidinium Bromide | 16 mg |
| Absolute Alcohol | 0.192 g |
| Lecithin | 0.0044 mg |
| Propellant HFA 134a | 9.40 g |

### Example 7

An aerosol formulation was prepared using formoterol and aclidinium or a salt thereof, preferably aclidinium bromide, with absolute alcohol, sorbitan trioleate, citric acid and a propellant, which is preferably HFA 134a. The amounts of the materials is preferably as shown in the table:

| Ingredients | Qty/Can |
|---|---|
| Formoterol | 0.96 mg |
| Aclidinium Bromide | 32 mg |
| Absolute Alcohol | 1.44 g |
| Sorbitan trioleate | 0.11 mg |
| Citric acid | For pH adjustment |
| Propellant HFA 134a | 8.16 g |

### Example 8

An aerosol formulation was prepared using formoterol and aclidinium or a salt thereof, preferably aclidinium bromide, with absolute alcohol, lecithin, hydrochloric acid, and a propellant, which is preferably HFA 134a. The amounts of the materials is preferably as shown in the table:

| Ingredients | Qty/Can |
|---|---|
| Formoterol | 0.96 mg |
| Aclidinium Bromide | 32 mg |
| Absolute Alcohol | 1.44 g |
| Lecithin | 0.11 mg |
| Hydrochloric acid | For pH adjustment |
| Propellant HFA 134a | 8.16 g |

### Example 9

An aerosol formulation was prepared using AR-formoterol and aclidinium or a salt thereof, preferably aclidinium bromide, with a propellant, which is preferably HFA P227. The amounts of the materials is preferably as shown in the table:

| Ingredients | Qty/Can |
|---|---|
| AR-formoterol | 0.48 mg |
| Aclidinium Bromide | 32 mg |
| Propellant HFA P227 | 11.2 gm |

### Example 10

An aerosol formulation was prepared using AR-formoterol and aclidinium or a salt thereof, preferably aclidinium bromide, with lactose and a propellant, which is preferably HFA 134a. The amounts of the materials is preferably as shown in the table:

| Ingredients | Qty/Can |
|---|---|
| AR-formoterol | 0.48 mg |
| Aclidinium Bromide | 32 mg |
| Lactose | 38 mg |
| Propellant HFA134a | 9.6 g |

### Example 11

An aerosol formulation was prepared using AR-formoterol and aclidinium or a salt thereof, preferably aclidinium bromide spray coated with PVP, with lactose and a propellant, which is preferably HFA P227. The amounts of the materials is preferably as shown in the table:

| Ingredients | Qty/Can |
|---|---|
| AR-formoterol | 0.48 mg |
| Aclidinium Bromide (spray coated with PVP) | 32 mg |
| Lactose | 38 mg |
| Propellant P227 | 11.2 g |

### Example 12

An aerosol formulation was prepared using AR-formoterol and aclidinium or a salt thereof, preferably aclidinium bromide, with PEG 100 and a propellant, which is preferably HFA 134a. The amounts of the materials is preferably as shown in the table:

| Ingredients | Qty/Can |
|---|---|
| AR-formoterol | 0.48 mg |
| Aclidinium Bromide | 16 mg |
| PEG 100 | 0.0288 g |
| Propellant HFA 134a | 9.6 g |

### Example 13

An aerosol formulation was prepared using AR-formoterol and aclidinium or a salt thereof, preferably aclidinium bromide, with absolute alcohol, oleic acid and a propellant, which is preferably HFA 134a. The amounts of the materials is preferably as shown in the table:

| Ingredients | Qty/Can |
|---|---|
| AR-formoterol | 0.48 mg |
| Aclidinium Bromide | 16mg |
| Absolute Alcohol | 1.44 g |
| Oleic acid | 0.11 mg |
| Propellant HFA134a | 8.16 g |

### Example 14

An aerosol formulation was prepared using AR-formoterol and aclidinium or a salt thereof, preferably aclidinium bromide, with absolute alcohol, lecithin and a propellant, which is preferably HFA 134a. The amounts of the materials is preferably as shown in the table:

| Ingredients | Qty/Can |
|---|---|
| AR-formoterol | 0.48 mg |
| Aclidinium Bromide | 16 mg |
| Absolute Alcohol | 0.192 g |
| Lecithin | 0.0044 mg |
| Propellant HFA 134a | 9.40 g |

### Example 15

An aerosol formulation was prepared using AR-formoterol and aclidinium or a salt thereof, preferably aclidinium bromide, with absolute alcohol, sorbitan trioleate, citric acid and a propellant, which is preferably HFA 134a. The amounts of the materials is preferably as shown in the table:

| Ingredients | Qty/Can |
|---|---|
| AR-formoterol | 0.48 mg |
| Aclidinium Bromide | 32 mg |
| Absolute Alcohol | 1.44 g |
| Sorbitan trioleate | 0.11 mg |
| Citric acid | For pH adjustment |
| Propellant HFA 134a | 8.16 g |

### Example 16

An aerosol formulation was prepared using AR-formoterol and aclidinium or a salt thereof, preferably aclidinium bromide, with absolute alcohol, lecithin, hydrochloric acid and a propellant, which is preferably HFA 134a. The amounts of the materials is preferably as shown in the table:

| Ingredients | Qty/Can |
|---|---|
| AR-formoterol | 0.48 mg |
| Aclidinium Bromide | 32 mg |
| Absolute Alcohol | 1.44 g |
| Lecithin | 0.11 mg |
| Hydrochloric acid | For pH adjustment |
| Propellant HFA 134a | 8.16 g |

### Example 17

An aerosol formulation was prepared using tiotropium or a salt thereof, preferably tiotropium bromide monohydrate, carmoterol, or a salt thereof, and ciclesonide, with lecithin and a propellant, which is preferably Propellant 11 and Propellant 12. The amounts of the materials is preferably as shown in the table:

| **Ingredients** | **Qty/can** |
|---|---|
| Tiotropium bromide monohydrate | 1.980 mg |
| carmoterol | 0.32 mg |
| ciclesonide | 0.16mg |
| Lecithin | 0.304 mg |
| Propellant 11 | 3.22 gms |
| Propellant 12 | 8.0 gms |

### Example 18

An aerosol formulation was prepared using tiotropium or a salt thereof, preferably tiotropium bromide monohydrate and carmoterol, or a salt thereof, with lecithin and a propellant, which is preferably Propellant 11 and Propellant 114 in combination with Propellant 12. The amounts of the materials is preferably as shown in the table:

| **Ingredients** | **Qty/can** |
|---|---|
| Tiotropium bromide monohydrate | 1.980 mg |
| carmoterol | 0.64 mg |
| Lecithin | 0.304 mg |
| Propellant 11 + Propellant 114 | 3.22 gms |
| Propellant 12 | 8.0 gms |

### Example 19

An aerosol formulation was prepared using carmoterol, or a salt thereof, and ciclesonide, with oleic acid and a propellant, which is preferably Propellant 11 and Propellant 12. The amounts of the materials is preferably as shown in the table:

| **Ingredients** | **Qty/can** |
|---|---|
| ciclesonide | 0.16 mg |
| carmoterol | 0.32 mg |
| Oleic acid | 0.304mg |
| Propellant 11 | 3.22 gms |
| Propellant 12 | 8.0 gms |

### Example 20

An aerosol formulation was prepared using tiotropium or a salt thereof, preferably tiotropium bromide monohydrate, carmoterol, or a salt thereof, and ciclesonide, with sorbitan trioleate and a propellant, which is preferably Propellant 11 and Propellant 12. The amounts of the materials is preferably as shown in the table:

| **Ingredients** | **Qty/can** |
|---|---|
| Tiotropium bromide monohydrate | 1.980 mg |
| carmoterol | 0.64 mg |
| ciclesonide | 0.16mg |
| Sorbitan trioleate | 0.304mg |
| Propellant 11 | 3.22 gms |
| Propellant 12 | 8.0 gms |

### Example 21

An aerosol formulation was prepared using tiotropium or a salt thereof, preferably tiotropium bromide monohydrate and carmoterol, or a salt thereof, with oleic acid and a propellant, which is preferably Propellant 11 and Propellant 114 in combination with Propellant 12. The amounts of the materials is preferably as shown in the table:

| **Ingredients** | **Qty/can** |
|---|---|
| Tiotropium bromide monohydrate | 1.980 mg |
| carmoterol | 0.32 mg |
| Oleic acid | 0.304mg |
| Propellant 11 + Propellant 114 | 3.22 gms |
| Propellant 12 | 8.0 gms |

### Example 22

An aerosol formulation was prepared using carmoterol, or a salt thereof, and ciclesonide, with sorbitan trioleate and a propellant, which is preferably Propellant 11 and Propellant 114 in combination with Propellant 12. The amounts of the materials is preferably as shown in the table:

| **Ingredients** | **Qty/can** |
|---|---|
| ciclesonide | 0.16 mg |
| carmoterol | 0.64 mg |
| Sorbitan trioleate | 0.304mg |
| Propellant 11 + Propellant 114 | 3.22 gms |
| Propellant 12 | 8.0 gms |

### Example 23

An aerosol formulation was prepared using tiotropium or a salt thereof, preferably tiotropium bromide monohydrate, carmoterol, or a salt thereof, and ciclesonide, and a propellant, which is preferably HFA 134a. The amounts of the materials is preferably as shown in the table:

| **Ingredients** | **Qty/can** |
|---|---|
| Tiotropium bromide monohydrate | 1.980mg |
| carmoterol | 0.32mg |
| ciclesonide | 0.16mg |
| HFA 134a | 8.0gms |

### Example 24

An aerosol formulation was prepared using carmoterol, or a salt thereof, and ciclesonide, with a propellant, which is preferably Propellant P227. The amounts of the materials is preferably as shown in the table:

| **Ingredients** | **Qty/can** |
|---|---|
| ciclesonide | 0.16mg |
| carmoterol | 0.64mg |
| P227 | 9.6gms |

### Example 25

An aerosol formulation was prepared using tiotropium or a salt thereof, preferably tiotropium bromide monohydrate and carmoterol, or a salt thereof, with ethanol and a propellant, which is preferably HFA 134a. The amounts of the materials is preferably as shown in the table:

| **Ingredients** | **Qty/can** |
|---|---|
| Tiotropium bromide monohydrate | 1.980mg |
| carmoterol | 0.32mg |
| Ethanol | 0.16gms |
| HFA134a | 8.0gms |

### Example 26

An aerosol formulation was prepared using tiotropium or a salt thereof, preferably tiotropium bromide monohydrate, carmoterol, or a salt thereof, and ciclesonide, with ethanol and a propellant, which is preferably Propellant P227. The amounts of the materials is preferably as shown in the table:

| **Ingredients** | **Qty/can** |
|---|---|
| Tiotropium bromide monohydrate | 1.980mg |
| carmoterol | 0.64mg |
| Ciclesonide | 0.16mg |
| Ethanol | 0.192gms |
| P227 | 9.6gms |

### Example 27

An aerosol formulation was prepared using tiotropium or a salt thereof, preferably tiotropium bromide monohydrate and carmoterol, or a salt thereof, with oleic acid, ethanol and a propellant, which is preferably Propellant P227. The amounts of the materials is preferably as shown in the table:

| **Ingredients** | **Qty/can** |
|---|---|
| Tiotropium bromide monohydrate | 1.980mg |
| carmoterol | 0.32mg |
| Oleic acid | 0.000616mg |
| Ethanol | 0.192gms |
| P227 | 9.6gms |

### Example 28

An aerosol formulation was prepared using carmoterol, or a salt thereof, and ciclesonide, with oleic acid, ethanol and a propellant, which is preferably HFA 134a. The amounts of the materials is preferably as shown in the table:

| **Ingredients** | **Qty/can** |
|---|---|
| ciclesonide | 0.16mg |
| carmoterol | 0.64mg |
| Oleic acid | 3.08mg |
| Ethanol | 0.192gms |
| HFA134a | 9.6gms |

### Example 29

An aerosol formulation was prepared using tiotropium or a salt thereof, preferably tiotropium bromide monohydrate, carmoterol, or a salt thereof, and ciclesonide, with lactose and a propellant, which is preferably HFA 134a. The amounts of the materials is preferably as shown in the table:

| **Ingredients** | **Qty/can** |
|---|---|
| Tiotropium bromide monohydrate | 1.98mg |
| carmoterol | 0.32mg |
| ciclesonide | 0.16mg |
| Lactose | 3.036mg |
| HFA134a | 8.0gm |

### Example 30

An aerosol formulation was prepared using carmoterol, or a salt thereof, and ciclesonide, with lactose and a propellant, which is preferably Propellant P227. The amounts of the materials is preferably as shown in the table:

| **Ingredients** | **Qty/can** |
|---|---|
| ciclesonide | 0.16mg |
| carmoterol | 0.64mg |
| Lactose | 3.036mg |
| P227 | 9.6gms |

### Example 31

An aerosol formulation was prepared using tiotropium or a salt thereof, preferably tiotropium bromide monohydrate and carmoterol, or a salt thereof, with magnesium stearate and a propellant, which is preferably Propellant P227. The amounts of the materials is preferably as shown in the table:

| **Ingredients** | **Qty/can** |
|---|---|
| Tiotropium bromide monohydrate | 1.98mg |
| carmoterol | 0.32mg |
| Magnesium stearate | 0.3035mg |
| P227 | 9.6gms |

### Example 32

An aerosol formulation was prepared using tiotropium or a salt thereof, preferably tiotropium bromide monohydrate, carmoterol, or a salt thereof, and ciclesonide, with magnesium stearate and a propellant, which is preferably HFA 134a. The amounts of the materials is preferably as shown in the table:

| **Ingredients** | **Qty/can** |
|---|---|
| Tiotropium bromide monohydrate | 1.98mg |
| carmoterol | 0.64mg |
| Ciclesonide | 0.16mg |
| Magnesium stearate | 0.3035mg |
| HFA134a | 8.0gms |

### Example 33

An aerosol formulation was prepared using carmoterol, or a salt thereof, and ciclesonide, with isopropyl myristate and a propellant, which is preferably Propellant P227. The amounts of the materials is preferably as shown in the table:

| **Ingredients** | **Qty/can** |
|---|---|
| Ciclesonide | 0.16mg |
| carmoterol | 0.32mg |
| Isopropyl myristate | 0.3035mg |
| P227 | 9.6gms |

### Example 34

An aerosol formulation was prepared using tiotropium or a salt thereof, preferably tiotropium bromide monohydrate and carmoterol, or a salt thereof, with isopropyl myristate and a propellant, which is preferably HFA 134a. The amounts of the materials is preferably as shown in the table:

| **Ingredients** | **Qty/can** |
|---|---|
| Tiotropium bromide monohydrate | 1.98mg |
| carmoterol | 0.64mg |
| Isopropyl myristate | 0.3035mg |
| HFA134a | 8.0gms |

According to the following examples 35 to 39, the present invention may be manufactured by convenient means wherein the process comprises of one or more drugs being mixed optionally with a pharmaceutically acceptable carrier and providing the ingredients in a suitable dry powder inhaler ready for inhalation..

### Example 35

An dry powder formulation was prepared using tiotropium or a salt thereof, preferably tiotropium bromide monohydrate, and carmoterol, or a salt thereof, preferably carmoterol hydrochloride in combination with lactose. The amount of the materials is preferably as shown in the table:

| Ingredients | Qty / unit (mg) |
|---|---|
| Carmoterol Hydrochloride | 0.0040 |
| Tiotropium bromide monohydrate | 0.0225 |
| Lactose | 24.9735 |
| Total | 25.0000 |

### Example 36

An dry powder formulation was prepared using carmoterol, or a salt thereof, preferably carmoterol hydrochloride in combination with ciclesonide and lactose. The amount of the materials is preferably as shown in the table:

| Ingredients | Qty / unit (mg) |
|---|---|
| Carmoterol Hydrochloride | 0.0040 |
| Ciclesonide | 0.2000 |
| Lactose | 24.7960 |
| Total | 25.0000 |

### Example 37

An dry powder formulation was prepared using tiotropium or a salt thereof, preferably tiotropium bromide monohydrate, and carmoterol, or a salt thereof, preferably carmoterol hydrochloride in combination with ciclesonide and lactose. The amount of the materials is preferably as shown in the table:

| Ingredients | Qty / unit (mg) |
|---|---|
| Carmoterol Hydrochloride | 0.0040 |
| Tiotropium bromide monohydrate | 0.0225 |
| Ciclesonide | 0.2000 |
| Lactose | 24.7735 |
| Total | 25.0000 |

### Example 38

An dry powder formulation was prepared using tiotropium or a salt thereof, preferably tiotropium bromide monohydrate, and formoterol, or a salt thereof, preferably formoterol fumarate dehydrate in combination with budesonide and lactose. The amount of the materials is preferably as shown in the table:

| Ingredients | Qty / unit (mg) |
|---|---|
| Formoterol fumarate dihydrate | 0.0120 |
| Tiotropium bromide monohydrate | 0.0225 |
| Budesonide | 0.8000 |
| Lactose | 24.1655 |
| Total | 25.0000 |

### Example 39

An dry powder formulation was prepared using tiotropium or a salt thereof, preferably tiotropium bromide monohydrate, and formoterol, or a salt thereof, preferably formoterol fumarate dehydrate in combination with budesonide and lactose. The amount of the materials is preferably as shown in the table:

| Ingredients | Qty / unit (mg) |
|---|---|
| Formoterol fumarate dihydrate | 0.0120 |
| Tiotropium bromide monohydrate | 0.0225 |
| Ciclesonide | 0.4000 |
| Lactose | 24.5655 |
| Total | 25.0000 |

According to the following examples 40 to 42, the present invention may be carried out by a process comprising placing a sterile active(s) in a suitable container, then, aseptically adding the sterile bulk of other ingredients to the container having the active(s) and mixing the above solution by sonication or magnetic stirring and finally adding the above mixture to the remaining bulk.

### Example 40

An inhalation solution formulation was prepared using tiotropium or a salt thereof, preferably tiotropium bromide monohydrate, and carmoterol, or a salt thereof, preferably carmoterol hydrochloride in combination with ciclesonide, Tween 20, sodium chloride and water. The amount of the materials is preferably as shown in the table:

| Ingredients | Quantity |
|---|---|
| Carmoterol Hydrochloride | 2.000 mcg |
| Tiotropium bromide monohydrate | 9.000 mcg |
| Ciclesonide | 200.000 mcg |
| Tween 20 | 0.005 %w/v |
| Sodium chloride | 0.9 %w/v |
| Water | q. s. to 2 ml |

### Example 41

An inhalation solution formulation was prepared using carmoterol, or a salt thereof, preferably carmoterol hydrochloride in combination with ciclesonide, Tween 20, sodium chloride and water. The amount of the materials is preferably as shown in the table:

| Ingredients | Quantity |
|---|---|
| Carmoterol Hydrochloride | 20.000 mcg |
| Ciclesonide | 2000.000 mcg |
| Tween 20 | 0.05 %w/v |
| Sodium chloride | 0.9 %w/v |
| Water | q. s. to 2 ml |

### Example 42

An inhalation solution formulation was prepared using tiotropium or a salt thereof, preferably tiotropium bromide monohydrate, and carmoterol, or a salt thereof, preferably carmoterol hydrochloride in combination with sodium chloride and water. The amount of the materials is preferably as shown in the table:

| Ingredients | Quantity |
|---|---|
| Carmoterol Hydrochloride | 2.000 mcg |
| Tiotropium bromide monohydrate | 9.000 mcg |
| Sodium chloride | 0.9 %w/v |
| Water | q. s. to 2 ml |

### Example 43

An aerosol formulation was prepared using tiotropium or a salt thereof, formoterol, and budesonide, in combination with lecithin and a propellant, which is preferably Propellant 11 and Propellant 12. The amount of the materials is preferably as shown in the table:

| **Ingredients** | **Qty/can** |
|---|---|
| Tiotropium | 1.98 mg |
| Formoterol | 0.96 mg |
| budesonide | 64 mg |
| Lecithin | 6.7 mg |
| Propellant 11 | 3.22 gms |
| Propellant 12 | 8.0 gms |

### Example 44

An aerosol formulation was prepared using tiotropium or a salt thereof, formoterol, and ciclesonide, in combination with lecithin and a propellant, which is preferably Propellant 11 and Propellant 12. The amount of the materials is preferably as shown in the table:

| **Ingredients** | **Qty/can** |
|---|---|
| Tiotropium | 1.98 mg |
| Formoterol | 0.96 mg |
| Ciclesonide | 16 mg |
| Lecithin | 6.7mg |
| Propellant 11 | 3.22 gms |
| Propellant 12 | 8.0 gms |

### Example 45

An aerosol formulation was prepared using tiotropium or a salt thereof, formoterol, and budesonide, in combination with a propellant, which is preferably HFA134a. The amount of the materials is preferably as shown in the table:

| **Ingredients** | **Qty/can** |
|---|---|
| Tiotropium | 1.98 mg |
| Formoterol | 0.96 mg |
| budesonide | 64 mg |
| HFA134a | 9.6 gms |

### Example 46

An aerosol formulation was prepared using tiotropium or a salt thereof, formoterol, and budesonide, in combination with lactose and a propellant, which is preferably HFA 227. The amount of the materials is preferably as shown in the table:

| **Ingredients** | **Qty/can** |
|---|---|
| Tiotropium | 1.98 mg |
| Formoterol | 0.96 mg |
| budesonide | 64 mg |
| Lactose | 16.7 mg |
| HFA227 | 11.2 gms |

### Example 47

An aerosol formulation was prepared using tiotropium or a salt thereof, formoterol, and ciclesonide, in combination with a propellant, which is preferably HFA 134a. The amount of the materials is preferably as shown in the table:

| **Ingredients** | **Qty/can** |
|---|---|
| Tiotropium | 1.98 mg |
| Formoterol | 0.96 mg |
| Ciclesonide | 16 mg |
| HFA134a | 9.6 gms |

### Example 48

An aerosol formulation was prepared using carmeterol, or a salt thereof, ciclesonide, in combination with lecithin and a propellant, which is preferably Propellant 11 and Propellant 12. The amount of the materials is preferably as shown in the table:

| **Ingredients** | **Qty/can** |
|---|---|
| Carmeterol | 0.16 mg |
| Ciclesonide | 16 mg |
| Lecithin | 1.6 mg |
| Propellant 11 | 3.22 gms |
| Propellant 12 | 8.0 gms |

### Example 49

An aerosol formulation was prepared using ciclesonide and formoterol, in combination with lecithin and a propellant, which is preferably Propellant 11 and Propellant 12. The amount of the materials is preferably as shown in the table:

| **Ingredients** | **Qty/can** |
|---|---|
| Ciclesonide | 16 mg |
| Formoterol | 0.96 mg |
| Lecithin | 1.7 mg |
| Propellant 11 | 3.22 gms |
| Propellant 12 | 8.0 gms |

### Example 50

An aerosol formulation was prepared using carmoterol or a salt thereof and ciclesonide, in combination with a propellant, which is preferably HFA 134a. The amount of the materials is preferably as shown in the table:

| **Ingredients** | **Qty/can** |
|---|---|
| Carmeterol | 0.16 mg |
| Ciclesonide | 16 mg |
| HFA134a | 4.8 gms |

### Example 51

An aerosol formulation was prepared using formoterol and ciclesonide, in combination with a propellant, which is preferably HFA 134a. The amount of the materials is preferably as shown in the table:

| **Ingredients** | **Qty/can** |
|---|---|
| Ciclesonide | 16 mg |
| Formoterol | 0.96 mg |
| HFA134a | 4.8 gms |

### Example 52

An aerosol formulation was prepared using formoterol and ciclesonide, in combination with ethanol, lecithin and a propellant, which is preferably HFA 227. The amount of the materials is preferably as shown in the table:

| **Ingredients** | **Qty/can** |
|---|---|
| Ciclesonide | 16 mg |
| Formoterol | 0.96 mg |
| Ethanol | 224 mg |
| Lecithin | 0.0034 mg |
| HFA227 | 11.0 gms |

It is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items.

It must be noted that, as used in this specification, the singular forms "a," "an" and "the" include plural references unless the context clearly dictates otherwise. Thus, for example, reference to "a propellant" includes a single propellant as well as two or more different propellants; reference to a "cosolvent" refers to a single cosolvent or to combinations of two or more cosolvents and the like.

It will be appreciated that the invention described above may be modified within the scope of the claims.

## Claims

1. A pharmaceutical combination comprising a combination of two or more bronchodilators for simultaneous or sequential administration.

2. The pharmaceutical combination according to claim 1, comprising a combination of at least two bronchodilators in combination with at least one corticosteroid.

3. The pharmaceutical combination according to claim 1 or 2, wherein the bronchodilators are selected from anticholinergic agents or beta adrenergic agonists or a combination thereof.

4. The combination according to any one of the previous claims, wherein at least one of the bronchodilators is a beta-agonist.

5. The combination according to claim 4, wherein the or each beta-agonist is selected from formoterol, AR-formoterol, fenoterol, carmoterol and indacaterol, or a pharmaceutically acceptable salt or ester thereof.

6. The combination according to any one of the previous claims, wherein at least one of the bronchodilators is an anticholinergic agent.

7. The combination according to claim 6, wherein the anticholinergic agent is selected from aclidinium, ipratropium, tiotropium and oxitropium, or a pharmaceutically acceptable salt or ester thereof.

8. The combination according to any one of claims 2 - 7, wherein the corticosteroid is selected from budesonide, ciclesonide, mometasone and beclomethasone, or a pharmaceutically acceptable salt or ester thereof.

9. The combination according to any one of claims 2 - 8, wherein the bronchodilators are carmoterol and tiotropium and the corticosteroid is ciclesonide, or a pharmaceutically acceptable salt or ester thereof.

10. The combination according to any one of claims 1 - 7, wherein the bronchodilators are tiotropium and carmoterol, or a pharmaceutically acceptable salt or ester thereof.

11. The combination according to any one of claims 2 - 8, wherein the bronchodilator is carmoterol and the corticosteroid is ciclesonide, or a pharmaceutically acceptable salt or ester thereof.

12. The combination according to any one of claims 1 - 7, wherein the bronchodilators are aclidinium and formoterol and/or AR-formoterol, or a pharmaceutically acceptable salt or ester thereof.

13. The combination composition according to any preceeding claim, wherein all the active materials and any excipients are formulated in a single pharmaceutical composition.

14. The combination composition according to any one of claims 1 - 13, formulated for use in a metered dose inhaler.

15. The combination composition according to any one of claims 1 - 13, formulated for use as a dry powder inhalation formulation.

16. The combination composition according to any one of claims 1 - 13, formulated for use as an inhalation solution.

17. A metered dose inhaler comprising a container, a pharmaceutical combination according to any one of claims 1 - 13, disposed within the container, and a valve for metering a dose of the pharmaceutical combination from the container.
